# EUROPEAN PATENT SPECIFICATION

(11) **EP 1 027 037 B1**
(45) Date of publication and mention of the grant of the patent: **03.12.2003**
(21) Application number: 98945336.0
(22) Date of filing: 18.09.1998
(51) Int. Cl.: A61K 9/20, A61K 9/48, A61K 47/38, A61K 31/66

(54) **PHARMACEUTICAL PREPARATION COMPRISING CLODRONATE AS ACTIVE INGREDIENT AND SILICIFIED MICROCRYSTALLINE CELLULOSE AS EXCIPIENT**
ARZNEIMITTEL, DAS CLODRONAT ALS WIRKSTOFF UND MIT SILICIUMDIOXID BEHANDELTE MIKROKRISTALLINE CELLULOSE ALS FÜLLSTOFF ENTHÄLT
PREPARATION PHARMACEUTIQUE COMPRENANT DU CLODRONATE EN TANT QUE PRINCIPE ACTIF ET DE LA CELLULOSE MICROCRISTALLINE SILICIFIEE EN TANT QU'EXCIPIENT

(30) Priority: 19.09.1997 FI 973733
(43) Date of publication of application: 16.08.2000
(73) Proprietor: Schering Oy, 20210 Turku (FI)
(72) Inventor: LEHTOLA, Veli-Matti, FIN-20810 Turku (FI); RANTALA, Eeva-Maria, Susanne, FIN-21530 Paimio (FI); RANTALA, Pertti, Tapani, FIN-20660 Littoinen (FI)
(74) Representative: Grew, Eva Regina
(86) International application number: FI9800735
(87) International publication number: WO99015155

(56) References cited:
- WO-A1-94/26310
- WO-A1-95/13054
- WO-A1-96/21429

## Description

The object of the present invention is a pharmaceutical preparation for oral use, especially a tablet, which as its active ingredient contains a pharmacologically acceptable salt of dichloromethylene bisphosphonic acid, i.e. a clodronate, especially disodium clodronate, and which as an excipient contains silicified microcrystalline cellulose. Further objects of the invention are a process for the manufacture of said pharmaceutical preparation, and the use of silicified microcrystalline cellulose for the manufacture of said pharmaceutical preparation.

Clodronate or the disodium salt of dichloromethylene bisphosphonic acid, tetrahydrate, is useful for instance in the treatment and prophylaxis of disorders of the calcium metabolism, such as bone resorption, hypercalcaemia and osteoporosis. Based on its ability to form a strong complex with a Ca²⁺-ion, clodronate removes excessive calcium from the circulation, prevents calcium phosphate from dissolving from the bone and/or acts via cell-mediated mechanisms.

Clodronate has previously been administered orally in the form of conventional compressed tablets or capsules. Such a tablet or capsule disintegrates in the stomach of the patient and releases the active agent, which in the acidic environment of the stomach is converted to the free acid form. As clodronic acid is relatively poorly absorbed, the bioavailability of the active agent will be low and consequently clodronate has to be administered in relatively large doses for a prolonged time. A problem with clodronate preparations has therefore been how to achieve a sufficiently high amount and concentration of the active agent in a capsule or tablet, without having to use capsule or tablet sizes which are unpleasantly large for the patient.

Another problem with clodronate preparations has been that it is very difficult to mix untreated clodronate raw material to a homogenous mixture with other excipients and active agents present in the preparation. For example EP 275 468 discloses a process wherein clodronate raw material and excipients are mixed dry, a granulating liquid is added, the mixture is wet granulated and the granulate is dried. Due to the properties of clodronate, the clodronate powder thus obtained is, however, inaccurate as regards its composition and obviously difficult to handle (sticky, very poor flow properties). It is thus very difficult in practice to mix it with other substances used in the preparation, as well as to further process it, wherefore, for instance, a relatively large amount of gliding agents is needed. From the homogenous raw powder an unhomogenous and poorly flowing product mass is then obtained, which affects also the accuracy of dosing of the final medicament.

The above mentioned problem relating to clodronate raw material has partly been solved by the process described in WO 95/13054, wherein clodronate is crystallized specifically as the disodium clodronate tetrahydrate which is subsequently dry granulated by compressing in such a way that the crystal structure of the disodium clodronate tetrahydrate is preserved. The process is said to lead to ready-to-use granules of uniform quality and good handling characteristics wherefore excipients are needed in considerably smaller amounts than in the previous methods. However, it does not solve the problems relating to the preparation of clodronate dosage forms by wet granulation.

Wet granulation is widely used in the pharmaceutical industry in the preparation of solid dosage forms due to the advantages it offers compared to dry granulation and direct compression. Usually the amount of excipients needed in wet granulation is less than that required for direct compression, and thus an acceptably sized tablet may be obtained. Wet granulation also provides the material to be compressed with better wetting properties and the particles comprising the resulting granulate with optimized particle size and shape. Also the amount of drug in the granules is approximately the same, and thus the content uniformity of the final preparation is generally improved.

Microcrystalline cellulose is a common excipient used in formulations which are wet granulated prior to tabletting. It is suitable not only for adding bulk to the finished product but also has additional features that facilitate pellet formation. Unfortunately the exposure of microcrystalline cellulose to moisture in the wet granulation process severely reduces the compressibility of this excipient. This is particularly problematic in cases where a pharmaceutical preparation with a high dose of the active agent, such as in the case of clodronate, is desired as the loss of compressibility of the microcrystalline cellulose means that a larger amount of this excipient is needed to obtain an acceptably compressed final product. This in turn adds bulk, making the final product more difficult to swallow and thus reducing patient compliance.

According to the invention it has now been discovered that it is possible to achieve oral dosage forms of clodronate with acceptable size and uniform quality, however, with sufficiently high amount and concentration of the active agent in the preparation. In the preparation process of the novel oral dosage form of clodronate it is possible to use not only dry granulation but also wet granulation and direct compression techniques. This is achieved if the pharmaceutical preparation is an oral dosage form comprising easily compactible silicified microcrystalline cellulose as an excipient.

Silicified microcrystalline cellulose used in the preparation according to the invention is microcrystalline cellulose which has been coprocessed with from about 0.1 to about 20 % silicon dioxide, SiO₂, based on the amount of microcrystalline cellulose. It is an agglomerate of microcrystalline cellulose and silicon dioxide in which the microcrystalline cellulose and silicon dioxide are in intimate association with each other. This means that the silicon dioxide has been integrated with the microcrystalline cellulose particles but there is no chemical interaction between the two materials. In practice this is achieved e.g. by spray-drying a suspension of microcrystalline cellulose and silicon dioxide.

The advantage of the use of silicified microcrystalline cellulose in clodronate preparations is overall improved functionality in terms of e.g. powder flow, compactibility, tablet strength and especially reduced friability. Solid dosage forms containing high load of clodronate are now obtainable by direct compression, dry granulation or wet granulation technique. The amount of the silicified microcrystalline cellulose which must be used in the preparation process to obtain an acceptable solid dosage form is substantially reduced, compared to the amount of usual microcrystalline cellulose which must be used for the same purpose. This naturally results in substantial reduction in tablet size. The solid clodronate preparations according to the invention are also of uniform quality and possess excellent disintegration and dissolution properties.

Extensive friability has been a problem especially with tablets containing clodronate. Extensive friability means that tablets are easily crumbled or split into pieces. Surprisingly, this problem can also be overcome by the use of silicified microcrystalline cellulose. A person skilled in the art would expect that the silicon dioxide in the silicified microcrystalline cellulose functions the opposite way when used in clodronate preparations, i.e. that it would decrease crushing strength and increase friability as gliding agents usually do.

However, one of the advantages of the use of silicified microcrystalline cellulose for the manufacture of clodronate preparations is that the silicon dioxide of the silicified microcrystalline cellulose may also function as a gliding agent while it also improves the properties of the microcrystalline cellulose.

In the process of preparing clodronate tablets containing silicified microcrystalline cellulose, it is also possible to first granulate clodronate (either by wet granulation or dry granulation technique) and then to mix the dry granules with silicified microcrystalline cellulose and, if desired, with other excipients before direct compression of the mixture into tablets. This process is technically very feasible and provides clodronate tablets with all the advantages mentioned above.

Further advantages of the use of silicified microcrystalline cellulose for the manufacture of clodronate preparations, especially clodronate tablets, are an increase in the production rate and, consequently, a technically and economically feasible production process. Tablets containing clodronate and usual microcrystalline cellulose can be formed into tablets only at very low rates compared to tablets containing clodronate and silicified microcrystalline cellulose. The use of silicified microcrystalline cellulose enables the production rates to be increased considerably without adversely affecting the quality of tablets, as is shown in Example 8.

If desired, also other excipients in addition to silicified microcrystalline cellulose may be used in the solid dosage forms according to the invention. These excipients are known to a person skilled in the art, and their use in the manufacture of clodronate preparations has been disclosed e.g. in EP 336 851, US 3,683,080 and US 4,234,645.

Consequently, the preparation according to the invention may further comprise conventional gliding agents and lubricants, such as stearic acid or its salts (Mg-, Ca-), talc, starch, or a mixture of two or more gliding agents. If desired, also additional colloidal silica may be added in addition to what is included in the silicified microcrystalline cellulose.

Filling agents (weight balancing agents) which may be used are for example lactose, starch or its derivatives, mannitol, glucose, saccharose, microcrystalline cellulose, or a mixture of two or more filling agents. Also natural or artificial flavouring and sweetening agents may be used.

If desired, also disintegrants can be added to the preparation. These are disintegrants generally known in the art, such as for example cross-linked sodium carboxymethylcellulose, starch or its derivatives, croscarmellose, crospovidone, or mixtures of two or more disintegrants.

By using certain excipients one can also regulate, if desired, whether a preparation is to decompose in the stomach or only later in the gastrointestinal tract, and also the dissolving rate. Thus the preparation can be coated with as such known film forming agents, which dissolve at the desired pH, such as for example with shellac, cellulose acetate phthalate, hydroxypropyl methylcellulose phthalate, polyvinyl acetate phthalate, cellulose acetate trimellitate or various acryl and methacryl acid derivatives. Film forming agents are known to a person skilled in the art and are commercially available.

The composition comprising clodronate and silicified microcrystalline cellulose is suitable for administration not only as a tablet but also as a number of different formulations. Thus it can for example be filled in capsules, or used as granules or a powder according to the methods generally known in the art, and further coated, if desired. Especially preferred are tablets and capsules.

The amount of clodronate in the drug delivery form according to the invention can vary within wide limits, e.g. from 10 to 95 % by weight, being typically 50 to 90 % by weight. The amount of silicified microcrystalline cellulose can vary e.g. from about 1 to about 50 % by weight, being typically from about 5 to about 25 % by weight. Preferably the preparation according to the invention comprises 60 to 80 % by weight of anhydrous disodium clodronate, about 8-20 % by weight of silicified microcrystalline cellulose, and 0.5-10 % other excipients such as lubricants and disintegrants.

The following examples illustrate the invention without limiting the same.

### Example 1

Tablets were prepared with the following composition per tablet:

| | |
|---|---|
| Disodium clodronate tetrahydrate 1000 mg responding anhydrous disodium clodronate | 800 mg |
| Silicified microcrystalline cellulose | 205 mg |
| Carmellose sodium | 22 mg |
| Stearic acid | 15 mg |
| Magnesium stearate | 8 mg |

The silicified microcrystalline cellulose used (Prosolv 90, Mendell, USA) had a 2 % w/w silicon dioxide concentration.

In the first stage of the tablet preparation, the dry granulated clodronate was moistened with stearic acid in ethanol and then dried at about 30 °C to a moisture content of appr. 18.5 - 20 % . The dried granules were then sieved through a 1.5 mm sieve. Thereafter the clodronate-stearic acid granules were mixed with carmellose sodium, silicified microcrystalline cellulose and magnesium stearate. The mixture was formed into tablets in a tabletting apparatus, using 9 x 20 mm punches to form tablets of a mean weight of 1177 mg (± 2.5 %) and of a suitable strength, for example 4 - 10 kg.

If desired, the prepared tablets may be coated with a coating solution, the composition of which per tablet may be for example the following:

| | |
|---|---|
| Methyl hydroxypropylcellulose phthalate | 42.8 mg |
| Diethyl phthalate | 6.4 mg |
| Ethanol | q. s. |
| Purified water | q.s. |

### Example 2

Tablets were prepared with the following composition per tablet:

| | |
|---|---|
| Disodium clodronate tetrahydrate 1000 mg responding anhydrous disodium clodronate | 800 mg |
| Silicified microcrystalline cellulose | 155 mg |
| Carmellose sodium | 22 mg |
| Stearic acid | 15 mg |
| Magnesium stearate | 8 mg |

The tablets were prepared essentially as described in Example 1, using the same kind of silicified microcrystalline cellulose as in Example 1.

### Example 3

Tablets were prepared with the following composition per tablet:

| | |
|---|---|
| Disodium clodronate tetrahydrate 1000 mg responding anhydrous disodium clodronate | 800 mg |
| Silicified microcrystalline cellulose | 155 mg |
| Carmellose sodium | 22 mg |
| Stearic acid | 15 mg |
| Magnesium stearate | 8 mg |

The silicified microcrystalline cellulose used (Prosolv 50, Mendell, USA) had a 2 % w/w silicon dioxide concentration. The tablets were prepared essentially as described in Example 1.

### Example 4

Tablets were prepared with the following composition per tablet:

| | |
|---|---|
| Disodium clodronate tetrahydrate 1000 mg responding anhydrous disodium clodronate | 800 mg |
| Silicified microcrystalline cellulose | 140 mg |
| Carmellose sodium | 22 mg |
| Stearic acid | 15 mg |
| Polyvinylpyrrolidone | 15 mg |
| Magnesium stearate | 8 mg |

The silicified microcrystalline cellulose used (Prosolv 90, Mendell, USA) had a 2 % w/w silicon dioxide concentration. The tablets were prepared essentially as described in Example 1, with the exception that stearic acid was dissolved in polyvinylpyrrolidone instead of ethanol.

### Example 5

Tablets were prepared with the following composition per tablet:

| | |
|---|---|
| Disodium clodronate tetrahydrate 1000 mg responding anhydrous disodium clodronate | 800 mg |
| Silicified microcrystalline cellulose | 125 mg |
| Carmellose sodium | 22 mg |
| Stearic acid | 15 mg |
| Magnesium stearate | 8 mg |

The tablets were prepared essentially as described in Example 1, using the same kind of silicified microcrystalline cellulose as in Example 1.

### Example 6

Tablets were prepared with the following composition per tablet:

| | |
|---|---|
| Disodium clodronate tetrahydrate 1000 mg responding anhydrous disodium clodronate | 800 mg |
| Silicified microcrystalline cellulose | 132 mg |
| Carmellose sodium | 22 mg |
| Stearic acid | 15 mg |
| Magnesium stearate | 8 mg |

The tablets were prepared essentially as described in Example 1, using the same kind of silicified microcrystalline cellulose as in Example 1.

### Example 7

Tablets were prepared with the following composition per tablet:

| | |
|---|---|
| Disodium clodronate tetrahydrate 1000 mg responding anhydrous disodium clodronate | 800 mg |
| Silicified microcrystalline cellulose | 165 mg |
| Carmellose sodium | 22 mg |
| Stearic acid | 15 mg |
| Magnesium stearate | 8 mg |

The silicified microcrystalline cellulose used (Prosolv 50, Mendell, USA) had a 2 % w/w silicon dioxide concentration. The tablets were prepared essentially as described in Example 1, using tabletting speeds as indicated in Table 1. The results from the measurements of crushing strength and friability are also shown in Table 1.

**Table 1.**

| Crushing strength and friability of tablets according to Example 7, prepared at different tabletting speeds | | |
|---|---|---|
| Tabletting speed | Crushing strength | Friability |
| 30 000 tablets/h | 16 kp | 0.11 % |
| 40 000 tablets/h | 18 kp | 0.20 % |

### Example 8

Tablets having the same composition as the tablets prepared in Example 6 were prepared at different tabletting speeds. For comparison, tablets were also prepared at different tabletting speeds with the following composition per tablet:

| | |
|---|---|
| Disodium clodronate tetrahydrate 1000 mg responding anhydrous disodium clodronate | 800 mg |
| Microcrystalline cellulose (Emcocel 50 M) | 132 mg |
| Carmellose sodium | 22 mg |
| Stearic acid | 15 mg |
| Magnesium stearate | 8 mg |

Crushing strength and friability of the obtained tablets were measured. The results are shown in Table 2.

**Table 2.**

| Crushing strength and friability of tablets containing silicified microcrystalline cellulose (A) and of tablets containing usual microcrystalline cellulose (B). Tablets were prepared at different tabletting speeds as indicated in Table 2. | | | | |
|---|---|---|---|---|
| Tabletting speed | Strength of tablets A | Strength of tablets B | Friability of tablets A | Friability of tablets B |
| 15 000 tabl/h | np | 13 kp | np | 3.0 % |
| 30 000 tabl/h | 18 kp | 11 kp | 0.39 % | 38.0 % |
| 50 000 tabl/h | 18 kp | * | 2.50 % | * |

| | | | | |
|---|---|---|---|---|
| np not performed | | | | |
| * could not be tabletted | | | | |

Tablets containing usual microcrystalline cellulose could not be tabletted using a higher tabletting speed than 30 000 tablets/h, because tablets would have broken up.

## Claims

1. Pharmaceutical preparation containing as an active agent a pharmacologically acceptable salt of dichloromethylene bisphosphonic acid, **characterized in that** it is an oral solid dosage form comprising silicified microcrystalline cellulose, obtained by coprocessing microcrystalline cellulose with from about 0.1 to about 20 % silicon dioxide, based on the amount of microcrystalline cellulose.

2. Preparation according to claim 1, **characterized in that** it comprises 5-25 % by weight of silicified microcrystalline cellulose.

3. Preparation according to claim 1, **characterized in that** it comprises
a) from about 60 to 80 % by weight of anhydrous disodium clodronate;
b) from about 8 to 20 % by weight of silicified microcrystalline cellulose; and
c) from about 0.5 to 10 % by weight of lubricants and/or disintegrants.

4. Preparation according to any one of the preceding claims, **characterized in that** it is a tablet or capsule.

5. Preparation according to any one of the preceding claims, **characterized in that** the salt of dichloromethylene bisphosphonic acid is the disodium salt.

6. Process for the manufacture of a pharmaceutical preparation according to claim 1 **characterized in that** a wet granulation technique is used.

7. Process for the manufacture of a pharmaceutical preparation according to claim 1, **characterized in that** a dry granulation technique is used.

8. Process for the manufacture of a pharmaceutical preparation according to claim 1, **characterized in that** a direct compression technique is used.

9. Use of silicified microcrystalline cellulose obtained by coprocessing microcrystalline cellulose with from about 0.1 to about 20 % silicon dioxide, based on the amount of microcrystalline cellulose, for the manufacture of a pharmaceutical preparation containing as an active agent a pharmacologically acceptable salt of dichloromethylene bisphosphonic acid.

## Patentansprüche

1. Arzneimittel, das als Wirkstoff ein pharmakologisch verträgliches Salz von Dichlormethylenbisphosphonsäure enthält, **dadurch gekennzeichnet, dass** es eine orale feste Dosierungsform ist, die silicifizierte mikrokristalline Cellulose umfasst, die durch gemeinsame Verarbeitung mikrokristalliner Cellulose mit etwa 0,1 bis etwa 20 % Siliciumdioxid, bezogen auf die Menge mikrokristalliner Cellulose, erhalten ist.

2. Mittel nach Anspruch 1, **dadurch gekennzeichnet, dass** es 5 - 25 Gew.-% silicifizierte mikrokristalline Cellulose umfasst.

3. Mittel nach Anspruch 1, **dadurch gekennzeichnet, dass** es umfasst:
a) etwa 60 bis 80 Gew.-% wasserfreies Dinatriumclodronat;
b) etwa 8 bis 20 Gew.-% silicifizierte mikrokristalline Cellulose; und
c) etwa 0,5 bis 10 Gew.-% Gleitmittel und/oder Sprengmittel.

4. Mittel nach einem der vorstehenden Ansprüche, **dadurch gekennzeichnet, dass** es entweder eine Tablette oder eine Kapsel ist.

5. Mittel nach einem der vorstehenden Ansprüche, **dadurch gekennzeichnet, dass** das Salz von Dichlormethylenbisphosphonsäure das Dinatriumsalz ist.

6. Verfahren zur Herstellung eines Arzneimittels nach Anspruch 1, **dadurch gekennzeichnet, dass** ein Nassgranulierverfahren verwendet wird.

7. Verfahren zur Herstellung eines Arzneimittels nach Anspruch 1, **dadurch gekennzeichnet, dass** ein Trockengranulierverfahren verwendet wird.

8. Verfahren zur Herstellung eines Arzneimittels nach Anspruch 1, **dadurch gekennzeichnet, dass** ein direktes Komprimierverfahren verwendet wird.

9. Verwendung von silicifizierter mikrokristalliner Cellulose, die durch gemeinsame Verarbeitung mikrokristalliner Cellulose mit etwa 0,1 bis etwa 20 % Siliciumdioxid, bezogen auf die Menge mikrokristalliner Cellulose, erhalten wird, zur Herstellung eines Arzneimittels, das als Wirkstoff ein pharmakologisch verträgliches Salz von Dichlormethylenbisphosphonsäure enthält.

## Revendications

1. Préparation pharmaceutique contenant en tant qu'ingrédient actif un sel pharmacologiquement acceptable de l'acide dichlorométhylènebisphosphonique, **caractérisée en ce qu'**elle se présente sous la forme d'un dosage solide pour voie orale comprenant de la cellulose microcristalline siliciée, obtenue par co-traitement de cellulose microcristalline avec une quantité d'environ 0,1 à environ 20 % de dioxyde de silicium, par rapport à la quantité de cellulose microcristalline.

2. Préparation suivant la revendication 1, **caractérisée en ce qu'**elle comprend 5-25 % en poids de cellulose microcristalline siliciée.

3. Préparation suivant la revendication 1, **caractérisée en ce qu'**elle comprend
a) environ 60 à 80 % en poids de clodronate disodique anhydre ;
b) environ 8 à 20 % en poids de cellulose microcristalline siliciée ; et
c) environ 0,5 à 10 % en poids de lubrifiants et/ou désintégrants.

4. Préparation suivant l'une quelconque des revendications précédentes, **caractérisée en ce qu'**elle se présente sous la forme d'un comprimé ou d'une gélule.

5. Préparation suivant l'une quelconque des revendications précédentes, **caractérisée en ce que** le sel de l'acide dichlorométhylènebisphosphonique est le sel disodique.

6. Procédé pour la fabrication d'une préparation pharmaceutique selon la revendication 1, **caractérisé en ce qu'**il comprend la mise en oeuvre d'une technique de granulation humide.

7. Procédé pour la fabrication d'une préparation pharmaceutique selon la revendication 1, **caractérisé en ce qu'**il comprend la mise en oeuvre d'une technique de granulation sèche.

8. Procédé pour la fabrication d'une préparation pharmaceutique selon la revendication 1, **caractérisé en ce qu'**il comprend la mise en oeuvre d'une technique de compression directe.

9. Utilisation de cellulose microcristalline siliciée obtenue par co-traitement de cellulose microcristalline avec une quantité d'environ 0,1 à environ 20 % de dioxyde de silicium, par rapport à la quantité de cellulose microcristalline, pour la fabrication d'une préparation pharmaceutique contenant en tant qu'ingrédient actif un sel pharmacologiquement acceptable de l'acide dichlorométhylènebisphosphonique.
